# EUROPEAN PATENT SPECIFICATION

(11) **EP 3 582 834 B1**
(45) Date of publication and mention of the grant of the patent: **11.11.2026**
(21) Application number: 18754262.6
(22) Date of filing: 15.02.2018
(51) Int. Cl.: A61M 5/31, A61M 5/315

(54) **SYRINGE AND PLUNGER ROD WITH STOPPER GUIDE**
SPRITZE UND KOLBENSTANGE MIT STOPFENFÜHRUNG
SERINGUE ET TIGE DE PISTON AVEC GUIDE DE BUTÉE

(30) Priority: 17.02.2017 US 201762460586 P
(43) Date of publication of application: 25.12.2019
(73) Proprietor: Becton, Dickinson and Company, Franklin Lakes, NJ 07417-1880 (US)
(72) Inventor: BOGERT, Gerald, Maywood New Jersey 07607 (US); MONDRO, Jason, Sparta New Jersey 07871 (US); PRAIS, Alfred, Hewitt New Jersey 07421 (US)
(74) Representative: dompatent
(86) International application number: PCT/US2018/018269
(87) International publication number: WO 2018/152269

(56) References cited:
- EP-A1- 2 298 392
- EP-A1- 2 298 392
- EP-A2- 2 684 576
- EP-A2- 2 684 576
- WO-A2-2008/136775
- WO-A2-2008/136775
- US-A1- 2011 034 882
- US-A1- 2013 110 044
- US-A1- 2014 155 826
- US-A1- 2014 155 826
- US-B1- 6 206 859
- US-B1- 6 206 859

## Description

This application claims priority to U.S. Provisional Application No. 62/460,586, filed February 17, 2017.

### FIELD OF THE INVENTION

The present invention relates generally to a syringe and a plunger rod. The invention is particularly directed to a plunger rod provided with a stopper guide for assisting in maintaining a seal and preventing leakage when the plunger rod is off-axis from the syringe barrel.

### BACKGROUND OF THE INVENTION

Syringes are used for delivering a substance and for withdrawing and collecting arterial blood samples from the body of a patient. Various types of syringes have been devised that include a plastic or glass syringe barrel, a sealing elastomeric stopper, and a plunger rod. The syringes can include a self-sealing filter that allows passage of air out of the syringe during collection of a sample, while still preventing the passage of fluid being collected. This type of syringe having a filter allows for a sample to be collected without the need to aspirate the syringe, as is required with a syringe having a plunger rod and a plunger stopper.

A standard syringe having a plunger rod with the stopper attached can be subjected to side load applied to the plunger rod. A side load or force applied at an inclined angle with respect to the direction of travel of the plunder rod can result in distortion of the stopper relative the syringe barrel that may result in leakage around the stopper.

Typical syringes include a two-piece plunger rod assembly comprised of an elastomeric sealing stopper attached to a plunger rod. U.S. Patent No. 5,314,416 to Lewis et al. discloses a low friction syringe assembly having a typical two-piece plunger rod and a plunger tip assembly. The sealing stopper and plunger rod are assembled together in a separate operation prior to assembly with a syringe barrel. A silicone lubricant is usually used on the interior wall of the syringe barrel to facilitate sliding movement of the elastomeric sealing stopper against the interior wall of the syringe barrel. A needle can be connected to the syringe to dispense the contents. The needle can also access a blood vessel for collecting a blood sample where the syringe is thereafter aspirated by the holding the syringe in a fixed position and drawing the plunger rearward within the syringe barrel to draw a blood sample into the syringe barrel.

After completion of the sample collection, the needle is removed and the syringe containing the collected sample is then transported to the laboratory. The plunger that is protruding from the syringe barrel can make handling and transportation of the syringe difficult and special care has to be taken not to dislodge the plunger that can cause leakage. When the plunger rod is withdrawn from the syringe barrel, the plunger rod may not be stable and can deflect to a position where the axis of the plunger rod is not aligned with the axis of the syringe barrel. The stopper can be deflected at an angle that can cause leakage.

While the prior devices have been generally suitable for the intended use, there is a continuing need in the industry for improved devices for collecting and delivering a substance to a patient.

WO 2008/136775 A2 describes a safety syringe comprises: a hollow barrel, a plunger with a piston, a hard rubber adaptor, and a needle. The piston in WO 2008/136775 A2 includes a rubber element having a central room space to allow the plunger tip to move axially therein for performing functions to enable drawing fluid starting at Occ level and interlocking respectively. In WO 2008/136775 A2, the adaptor affixed at a distal protruding nose of the barrel and the needle is locked at a threaded portion within the adaptor snugly, which includes a female interlocking structure at proximal end to receive a male interlocking structure aligning a loosen means of the plunger, characterized in that the male interlocking structure is loose staying within, carrying the adaptor with the needle into the barrel upon retracting the plunger, in the manner that the loosen adaptor declines together with the needle causing the needle tip declined and is abutted by the base within. In WO 2008/136775 A2, further pulling the plunger, the piston is abutted by the abutting structure of the barrel allowing the plunger to break at a fragile portion provided, thereby absolutely ensuring non-reusable and avoid needle stick injuries.

EP 2 684 576 A2 describes a syringe including a barrel and a plunger slidably disposed within the barrel is disclosed. In EP 2 684 576 A2, a distal end of the barrel defines a roof, and an elongate tip, in fluid communication with the barrel, extends from the roof. In EP 2 684 576 A2, a protrusion forming a ridge is disposed on one of the roof of the barrel and the plunger head. When the plunger head in EP 2 684 576 A2 contacts the roof, the protrusion forms a channel between the plunger head and the roof. The channel in EP 2 684 576 A2 isolates fluid within the channel from the elongate tip, which in turn reduces the spray of fluid through the distal end of the syringe.

US 2014/155826 A1 describes syringe assemblies having a passive disabling system to prevent reuse are provided. According to one or more embodiments of US 2014/155826 A1, the syringe assembly comprises a barrel, plunger rod and detachable thumb press wherein the plunger rod further comprises flexible locking petals that lock the plunger rod within the barrel and one or more frangible connection or break point at the proximal end of the plunger rod disposed either inside the thumb press or immediately adjacent to the thumb press to separate the thumb press from the main body of the plunger rod when a user applies sufficient proximal force to the plunger rod after it has been locked within the barrel.

EP 2 298 392 A1 describes an injector in which a backstop function is realized by a collar part at a position situated away from a gasket in the direction of the side of the proximal end and adequate sterilization can be carried out, with a plunger rod (in which the collar part is formed) assembled, while aiming to secure airtightness.

US 6 206 859 B1 describes a self-aligning reinforced plunger rod and a non-elastomeric plastic plunger insert assembly for use with an elastomeric plunger in a cartridge or syringe barrel, the plunger rod having a first, second, third and fourth sides, a distal end with an end disc, a proximal end with a thumb rest, a central reinforcing disc at the midpoint between the end disc and the thumb rest, and a plunger rod tip integral with the end disc and having a neck portion and a ball portion for engagement with a cavity in the non-elastomeric plastic plunger insert. In US 6 206 859 B1, connecting the end disc and thumb rest there are: four longitudinal radially extending rectangular vanes spaced about 90° from each other; on the first side of the plunger rod a first pair of longitudinal radially extending reinforcing vanes, and a second pair of longitudinal radially extending reinforcing vanes; on the third side opposite to the first side of the plunger rod, a first pair of longitudinal radially extending reinforcing vanes, and a second pair of longitudinal radially extending reinforcing vanes; each of the first pair and each of the second pair of longitudinal radially extending reinforcing vanes on the first side of the plunger rod and each of the first pair and each of the second pair of the longitudinal radially extending reinforcing vanes on the third side of the plunger rod forms an obtuse triangle with one of the longitudinal radially extending reinforcing vanes; on the second side of the plunger rod, a first pair of longitudinal radially extending reinforcing vanes forms a two-dimensional hourglass-shape surface, and on the fourth side of the plunger rod, a second pair of longitudinal radially extending reinforcing vanes forms a two-dimensional hourglass-shape surface. In US 6 206 859 B1, the non-elastomeric plastic plunger insert contains a cavity defined by its inside surface to receive the plunger rod tip. In US 6 206 859 B1, the combination directs the force exerted on the plunger rod in an axial direction.

### SUMMARY OF THE INVENTION

Objects of the invention are achieved with a plunger rod configured for use with a syringe barrel having an open proximal end and an axial passage as defined in claim 1. The present invention is defined in claim 1. If any embodiments are described in the present disclosure that are not encompassed by claim 1, they are to be understood as examples intended to support understanding.

One embodiment of the invention is directed to a plunger rod for dispensing and delivering a substance to a patient or for collecting a sample from patient. The invention is particularly directed to a plunger rod that reduces the incidence of leakage between the stopper and the syringe barrel.

A syringe in one example has a syringe barrel with an open end for receiving a plunger rod and an outlet end for dispensing or collecting a fluid. The plunger rod has a stopper coupled to one end of the plunger rod for sliding within the syringe barrel to dispense or collect the fluid. The plunger rod is configured to slide within the syringe barrel while maintaining a stable axial position within the syringe barrel.

One aspect of the present invention is to provide a plunger rod having an annular plate forming a rib spaced from the distal end of the rod and spaced from the stopper that cooperates with the inner surface of the syringe barrel to stabilize and maintain the axis of the plunger rod on the axis of the syringe barrel.

A further aspect of the invention is to provide a plunger rod for supporting a stopper that slides within a syringe barrel where the plunger rod has two or more axially spaced points of support that can complement or engage the inner surface of the syringe barrel. In one embodiment, two or more plates or ribs are spaced apart a distance to limit tilting of the plunger rod off axis relative to the axis of the syringe barrel thereby preventing distortion of the stopper. The two spaced apart points of support maintain the longitudinal axis of the plunger rod and the longitudinal axis of the stopper parallel to the longitudinal axis of the syringe barrel.

The foregoing and/or other aspects of the present invention are achieved by providing a plunger rod for a syringe where the plunger rod has a longitudinally extending shaft with a distal first end for coupling to a stopper, and a proximal second end opposite the distal end for operating the plunger rod. An annular plate forming a rib is positioned at the first end and oriented to engage the inner surface of a syringe barrel during sliding of the plunger rod within the syringe barrel to maintain the axis of the plunger rod parallel to the longitudinal axis of the syringe barrel. By maintaining the plunger rod on the axis of the syringe barrel, the plunger remains on the axis of the syringe barrel to reduce the occurrence of leakage.

The features of the present invention are achieved by providing a syringe assembly having a syringe barrel, a plunger rod and a stopper coupled to the distal end of the plunger rod. The plunger rod has a shaft with a distal end and a proximal end. A coupling member extends from the distal end for supporting the stopper. At least one support is positioned at the distal end for cooperating with the syringe barrel to limit tilting of the plunger rod relative to the syringe barrel.

The various features of the present invention are also achieved by providing a plunger rod with at least two spaced apart supports providing at least two supporting points for the plunger rod and supporting the position of the plunger rod within the syringe barrel during use. The shaft includes a base plate at the distal end forming a first support point and for mating with and supporting the stopper. The second support point is provided by a support spaced axially from the based plate a distance to limit tilting of the plunger rod within the syringe barrel. In one embodiment, the support is a support plate or an outwardly extending projection.

The features of the invention are further attained by providing a syringe assembly including a syringe barrel and a plunger rod. The syringe barrel has an axial dimension with an axial passage with an open end. The plunger rod has a shaft with a first end with a stopper coupled to the first end for sliding in the syringe barrel. The shaft of the plunger rod has a second end for actuating the plunger rod by the user. At least one rib extends outward from the shaft to engage the inner surface of the syringe barrel when the plunger rod is tilted relative to the axis of the syringe barrel.

The features of the invention provide at least one support, such as a support plate, extending outward from the plunger shaft where the support plate is spaced from the first end a distance to stabilize and resist tilting of the plunger rod when the plunger rod is withdrawn toward the open end of the syringe barrel.

A syringe assembly in one embodiment includes a syringe barrel having an open end and an axial passage and a plunger rod. The plunger rod includes a shaft having a longitudinal axis with a first end and a second end opposite said first end. A stopper support is provided at the first end forming a first contact point with an inner surface of said syringe barrel. A coupling member is provided on the stopper support at the first end and a stopper is coupled to the coupling member. The stopper has an outer dimension for sliding in the axial passage of said syringe. The shaft has at least one plunger rod support extending radially outward from the shaft and spaced from the first end forming at least one second contact point with the inner surface of the syringe barrel. The at least one plunger rod support has an outer dimension for engaging the inner surface of the syringe barrel to resist lateral movement and tilting of the plunger rod relative to the syringe barrel. The plunger rod support is spaced from the stopper support a distance of about 1 to 1.5 times an inner diameter of the syringe barrel.

Additional and/or other aspects and advantages of the present invention will be set forth in part in the description that follows and, in part, will be apparent from the description, or by practicing the invention.

### BRIEF DESCRIPTION OF THE DRAWINGS

The above and/or other aspects and advantages of embodiments of the invention will be more readily appreciated from the following detailed description, taken in conjunction with the accompanying drawings, in which:
Fig. 1 is a side view in cross section of a standard syringe plunger rod assembly;
Fig. 2 is a cross sectional side view showing the syringe assembly of the invention where the plunger rod is received in the syringe barrel;
Fig. 3 is a cross-sectional view showing the plunger rod retracted to a position toward the open end of the syringe barrel;
Fig. 4 is a partial cross-sectional view illustrating plunger rod and stopper;
Fig. 5 is a front perspective side view illustrating the plunger rod;
Fig. 6 is a front perspective view of the plunger rod;
Fig. 7 is a rear perspective view of the plunger rod;
Fig. 8 is a front perspective view of a coupling end of the plunger rod;
Fig. 9 is a rear perspective view of the coupling end of the plunger rod;
Fig. 10 is an enlarged side view of coupling end of the plunger rod; and
Fig. 11 is an end view of the plunger rod taken along line 11-11 of Fig. 10.

### DETAILED DESCRIPTION OF EXEMPLARY EMBODIMENTS

Reference is made in detail to embodiments of the present invention, examples of which are illustrated in the accompanying drawings, wherein like reference numerals refer to the like elements throughout. The descriptions of these embodiments exemplify the present invention by referring to the drawings.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are exemplary embodiments of the invention. Specific dimensions and other physical characteristics related to the embodiments disclosed herein are not to be considered as limiting. The embodiments are not intended to be mutually exclusive so that the features of one embodiment can be combined with other embodiments.

As used herein, the term "proximal" refers to a location assembly according to the embodiments of this invention that, during normal use, is closest to the clinician using the device and farthest from the patient in connection with whom the device is used. Conversely, the term "distal" refers to a location on the assembly of this invention that, during normal use, is farthest from the clinician using the device and closest to the patient in connection with whom the device is used. Furthermore, the term "proximal direction" indicates a direction of movement away from the patient and toward the user of the blood collection assembly, whereas the term "distal direction" indicates a direction of movement away from the user of the assembly and toward the patient.

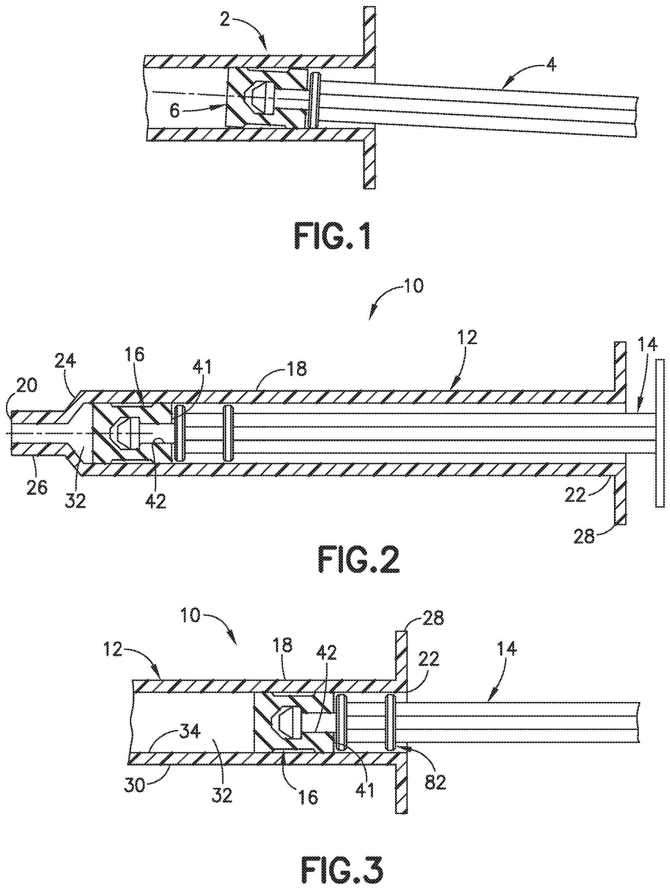

Fig. 1 is side view in cross section of a standard syringe barrel 2 and a plunger rod 4 having a stopper at one end. The plunger rod of Fig. 1 has an outer dimension that is slightly less than the inner dimension of the syringe barrel to be able to slide axially within the syringe barrel. As a result, the lateral force applied to the plunger rod causes the plunger rod and the stopper to move to a position that are not parallel to the axis of the syringe barrel where the stopper can distort and cause leakage around the stopper. When the plunger rod is retracted to a position shown in Fig. 1 within the axial passage of syringe barrel 2, the plunger rod can tilt off axis relative to the axis of syringe barrel 2. This can result in ribs of the stopper compressing or distorting on one side as shown resulting in leakage around stopper 6. The ribs can also be lifted off contact with the inner surface of syringe barrel 2 when a side load is applied to plunger rod 4. The pressure within the syringe barrel can then result in leakage around the ribs of the stopper when the stopper is tilted off-axis.

Referring to Figs. 2-11, the syringe assembly 10 of the invention includes a syringe barrel 12, a plunger rod 14, and stopper 16 coupled to the plunger rod 14. The syringe barrel 12 can be a standard syringe barrel having a substantially cylindrical body 18 with a distal first end 20 defining an outlet end and a proximal second end 22 defining an open end for receiving the plunger rod and stopper. The outlet end in the embodiment shown has a conical shaped end portion 24 that converges to an outlet nozzle 26. The nozzle 26 can receive and support a cannula or can be coupled to a suitable delivery device for delivering the contents of the syringe to a patient. In other embodiments, nozzle 26 can be a Luer fitting.

The open end 22 includes a flange 28 for manipulating syringe barrel 12 in a standard manner. The syringe barrel 12 is formed with a side wall 30 having an axial passage 32 defining an inner chamber for containing the substance to be delivered by sliding plunger rod 14 and stopper 16 toward first open end 20. The axial passage 24 is formed with a smooth inner surface 34. In the embodiment shown, barrel 12 has a substantially cylindrical shaped side wall 30. A suitable lubricant, such as a silicone lubricant, can be applied to the inner surface to assist in the sliding of the stopper within the syringe barrel. The syringe barrel can be made of glass or a suitable rigid clear plastic material. The plunger rod can also be made of a rigid plastic material.

The syringe assembly 10 of the invention is constructed to reduce the incidence of leakage between stopper 16 and the inner surface of the syringe barrel during use. The plunger rod 16 is configured to maintain the plunger rod on the intended axis and in line with the axis of the syringe barrel. The plunger rod 16 includes at least one and typically two points of support with respect to the syringe barrel to limit the tilting of the plunger rod with respect to the syringe barrel and limit distortion of the stopper.

As shown in Figs. 2-4, stopper 16 is coupled to plunger rod 14 to slide within the axial passage 32 of the syringe barrel 12 and maintain contact with the inner surface 34 to provide a seal sufficient to dispense the contents of the syringe without leakage between the stopper and the syringe barrel. The shape of stopper 16 provides sufficient contact surface area to form a suitable seal while limiting the contact surface to enable the stopper to slide easily within the syringe barrel. A large surface area on the stopper in contact with the inner surface of the syringe barrel can provide a high friction and interference such that sliding of the stopper becomes difficult under normal use.

In the embodiment show, stopper 16 has a substantially cylindrical shape with an outer annular surface 36, an outer end 38, and a recess 40 at an inner end 41 opposite the outer end 38. The outer end 38 provides an end surface for contacting the contents of the syringe and dispensing the contents from the syringe barrel. Recess 40 is formed with an annular lip 42 that extends radially inward for coupling to plunger rod 14.

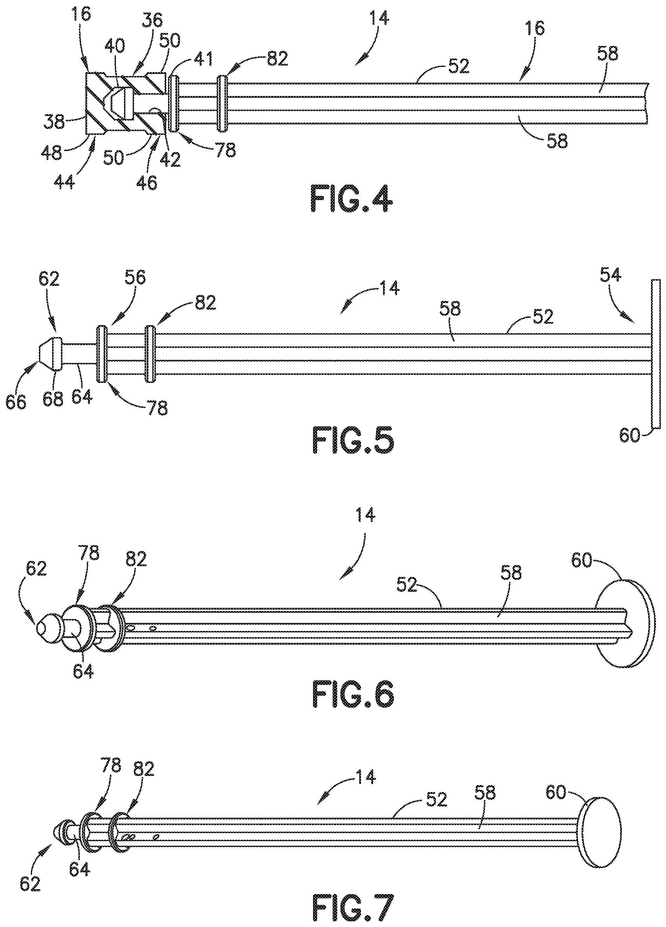

Outer surface 36 of stopper 16 in the embodiment shown in Figs. 3 and 4 has a leading rib 44 and trailing rib 46 for contacting the inner surface 34 of syringe barrel 12. Ribs 44 and 46 have a substantially annular shape and extend radially outward from the body of stopper 16. Ribs 44 and 46 have outer annular faces 48 and 50, respectively, for contacting the inner surface 34 of syringe barrel 12 and forming a seal. The outer annular faces in the embodiment shown have a substantially cylindrical shape to provide a sufficient contact area with syringe barrel 12. The annular shaped ribs 44 and 46 are spaced apart a distance to provide stability to the stopper during movement and use and reduce the incidence of the stopper twisting or tilting and maintaining the axis of the stopper oriented parallel and coaxial to the axis of the syringe barrel. In one embodiment, a lubricant can be provided on the inner surface of the syringe barrel to assisting in sliding movement of the stopper with respect to the syringe barrel. The stopper can be made of a suitable elastomeric material, such as isoprene rubber, as known in the art.

Referring to Figs. 5-11, plunger rod 14 has a longitudinal shaft 52 with a proximal end 54 and distal end 56. Shaft 52 is formed with outwardly extending ribs 58 to provide strength and resist bending and deflection during use while limiting the amount of material needed to form the shaft. In one embodiment, four radially extending ribs 58 are provided to form a shape for providing sufficient strength to the shaft. A proximal end 54 is provided with an operating flange 60 for the operator during use. Ribs 58 extend outwardly a distance to define an outer dimension of shaft 52 that is slightly less than an inner diameter of syringe barrel 12 so that the shaft can slide within the syringe barrel without interference or friction that would inhibit the sliding movement of the plunger rod 14 during use. In one embodiment, ribs 58 extend the length of shaft 52.

Distal end 56 of shaft 52 includes a coupling member 62 for receiving and supporting stopper 16. The coupling member 62 includes a post 64 and an enlarged head 66 for mating with stopper 16. As shown in Fig. 5, post 64 has a diameter less than the outer dimension of shaft 52 and extends in an axial direction from the distal end 56. The head 66 protrudes from the axial end of post 64 and has a diameter larger than the diameter of post 64.

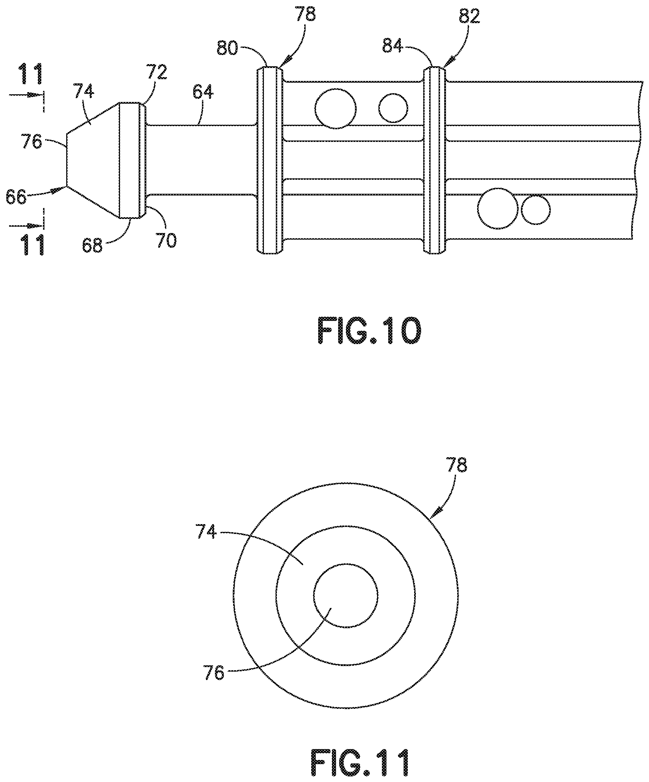

The head 66 has an outer annular face 68 and a bottom face 70 extending radially outward from post 64 to form a ledge 72 with outer annular face 68 for coupling with the stopper 16. The axial end of the head 66 has a conical shaped portion 74 converging to a substantially flat axial face 76 as shown in the end view of Fig. 11 and the side view of Fig. 10. As shown in Fig. 4, recess 40 of stopper 16 receives head 66 where annular lip 42 of stopper 16 engages ledge 72 and is retained on head 66.

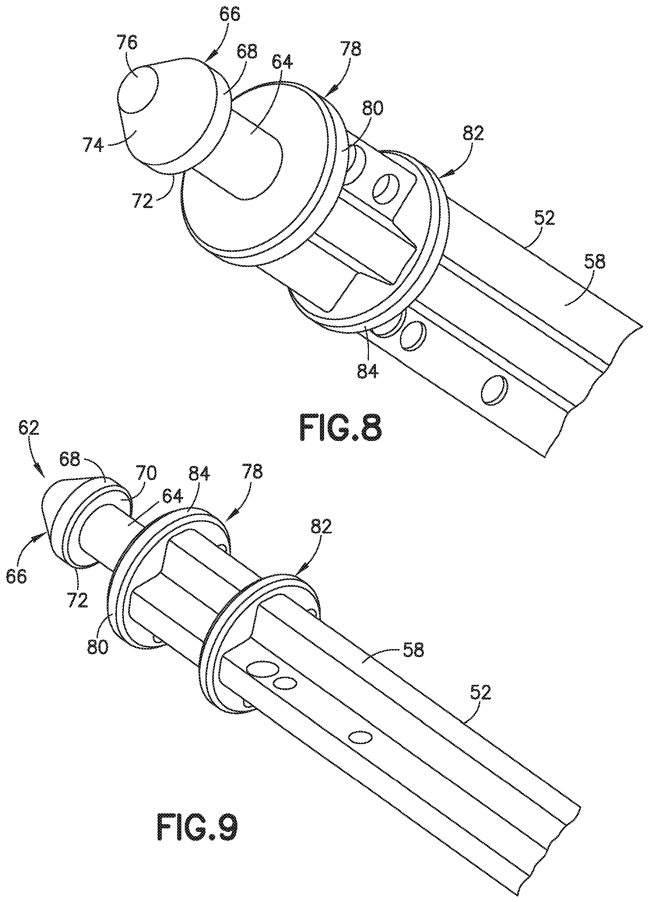

The shaft 52 includes a base plate 78 forming a push plate at distal end 56 at the base of post 64 so that post 64 extends axially from plate 78. Base plate 78 has a substantially circular shape with an outer annular edge 80 and an axial thickness to provide sufficient support for stopper 16 during the forward movement of plunger rod 14 in use. The outer annular edge 80 provides a first support point for plunger rod 14 and stopper 16 relative to syringe barrel 12. As shown in Fig. 4, base plate 78 has an outer dimension slightly less than an outer dimension of stopper 16 and a diameter slightly less than the inner diameter of syringe barrel 12 to enable sliding within syringe barrel 12 and supporting the plunger rod and stopper during use. The base plate 78 has an outer dimension to slide within axial passage 32 of syringe barrel 12 without interfering with the movement of plunger rod 14. As shown in Fig. 9, base plate 78 has an outer dimension greater than the outer dimension of ribs 58 of shaft 52.

A support 82 is formed on shaft 52 and spaced axially from base plate 78 a distance to stabilize the plunger rod when received in syringe barrel 12 and limit tilting of the shaft off axis from the axis of the syringe barrel when a side load is applied. In the embodiment shown, the support member 82 has a circular shape corresponding to the inner shape and dimension of the inner surface of the syringe barrel. The support can have other shapes that are able to contact the inner surface of the syringe barrel and support the plunger rod. In an alternative embodiment, the support can be a plurality of prongs or ribs spaced apart a distance to support the plunger rod. The support is spaced from the base plate a distance equal to or greater than the inner diameter of the syringe barrel.

In the embodiment shown, the support is a support plate 82 extending radially outward from the shaft 52 and has an outer radial outer dimension substantially the same as base plate 78. The outer annular edge 84 of support plate 82 is substantially coaxially aligned with the outer annular edge of base plate 78. Support plate 82 has an outer diameter slightly less than the inner diameter of syringe barrel 12 to allow sliding movement and maintain support of plunger rod 14 within the syringe barrel.

The outer diameter of the base plate and support plate has a nominal diameter less than the inner diameter of the syringe barrel for sliding and supporting the plunger rod on the axis of the syringe barrel when a side load is applied to the plunger rod. The axial spacing between the base plate and support plate is sufficient to contact the inner surface of the syringe barrel during normal use of the syringe. The spacing is selected so that the support plate is retained within the syringe barrel when the syringe is filed with contents prior to dispensing the contents to stabilize the plunger rod within the syringe barrel. The support plate is oriented relative to the base plate so that support plate is positioned within the syringe barrel throughout the normal operating position of the plunger rod with respect to the syringe barrel.

The spacing between the base plate and support plate can vary depending on the inner diameter of the syringe barrel and the axial length of the plunger rod and the axial length of the stopper. The spacing between the base plate and the support plate is a distance that prevents distortion of the stopper that can otherwise cause leakage. In one embodiment, the spacing between the base plate and the support plate can be equal to or greater than an inner diameter of the syringe barrel. Typically, the axial spacing between the base plate and the support plate is not less than an inner diameter of the syringe barrel. The spacing between the base plate and the support plate relative to the inner diameter of the syringe barrel in one embodiment can be about 1:1 to 2:1. In one embodiment, the ratio of the axial spacing between the base plate and support plate relative to the inner diameter of the syringe barrel is about 1:1 to about 1.5:1. In another embodiment, the axial spacing between the base plate and the support plate is about 1:1 with respect to the inner diameter of the syringe barrel so that the axial spacing is about equal to the inner diameter of the syringe barrel. In a further embodiment, the spacing between the base plate and the support plate is about 1 to 1.5 times the inner diameter of the syringe barrel.

As shown in Figs. 2 and 3, plunger rod 14 has a length complementing the axial length of the chamber of syringe barrel 12 for dispensing the contents of the syringe assembly 10. Fig. 2 shows plunger rod 14 in the forward position relative to the syringe barrel 12 either before filling the syringe or after dispensing the contents of syringe 12. As shown in Fig. 2, ribs 44 and 46 of stopper 16 contact inner surface 34 of syringe barrel 12 to form a fluid-tight seal for dispensing the contents of the syringe and preventing leakage through open end 22.

Fig. 3 shows plunger rod 14 and stopper 16 in a retracted position relative to the syringe barrel. As shown, base plate 78 and support plate 82 stabilize plunger rod 14 and prevent deflection or tilting of plunger rod 14 with respect to syringe barrel 12 thereby maintaining stopper 16 in a position axially aligned with the axis of syringe barrel 12 to prevent leakage that can occur when the stopper is tilted off-axis. During use, the forward force applied to the plunger rod by the user can result in a force being directed at an inclined angle with respect to axis of the syringe barrel that cause the stopper to twist or tilt off-axis. The plates 78 and 82 are provided with minimal clearance between the inner surface of the syringe barrel to resist tilting of the plunger rod and resist deflection or distortion of the stopper during use.

Although only a few exemplary embodiments of the present invention have been described in detail above, those skilled in the art will readily appreciate that many modifications are possible in the exemplary embodiments without materially departing from the novel teachings and advantages of this invention. Accordingly, all such modifications are intended to be included within the scope of the appended claims.

## Claims

1. A plunger rod configured for use with a syringe barrel having an open proximal end and an axial passage, said plunger rod comprising:
a shaft (52) having a longitudinal axis with a first end and a second end opposite said first end, said shaft having a plurality of outwardly extending ribs (58);
a coupling member (62) at said first end having a post with an enlarged head for coupling to a stopper (16), said coupling member having a base plate configured for supporting a proximal end of the stopper, and said base plate having an annular shape extending radially outward from said shaft;
**characterized in that**
said shaft having at least one support plate (82) with an annular shape and an outer diameter complementing and substantially the same as an outer diameter of said base plate and extending radially outward from said shaft, and having an outer dimension slightly less than an inner diameter of the syringe barrel for providing minimal clearance between an inner surface of the syringe barrel to stabilize the plunger rod when received in the syringe barrel, said support plate spaced axially from said base plate toward said second end a distance to stabilize and resist tilting and lateral movement of said plunger rod relative to the syringe barrel during movement of said plunger rod in a proximal direction with respect to the syringe barrel, and to inhibit leakage around said stopper when said syringe barrel is filled with a substance and said plunger rod is in a proximal position before dispensing the substance.

2. The plunger rod according to claim 1, wherein the syringe barrel has an internal diameter, and where said support plate is spaced from said base plate a distance of about 1 to 1.5 times the inner diameter of the syringe barrel.

3. The plunger rod of claim 1, wherein the axial spacing between the base plate and the support plate is 1:1 to 2:1 relative to the inner diameter of the syringe barrel.

4. The plunger rod of claim 1, wherein said support plate is spaced from said base plate a distance where said support plate contacts the inner surface of the syringe barrel when the syringe barrel is filled with contents prior to dispensing.

## Patentansprüche

1. Kolbenstange, die zur Verwendung mit einem Spritzenzylinder ausgebildet ist, der ein offenes proximales Ende und einen axialen Durchgang aufweist, wobei die Kolbenstange aufweist:
einen Schaft (52), der eine Längsachse mit einem ersten Ende und einem dem ersten Ende gegenüberliegenden zweiten Ende aufweist, wobei der Schaft eine Vielzahl von sich nach außen erstreckenden Rippen (58) aufweist;
ein an dem ersten Ende angeordnetes Kopplungselement (62), das einen Stift mit einem vergrößerten Kopf zum Koppeln mit einem Stopfen (16) aufweist, wobei das Kopplungselement eine Grundplatte aufweist, die zum Stützen eines proximalen Endes des Stopfens ausgebildet ist, und wobei die Grundplatte eine Ringform aufweist, die sich von dem Schaft radial nach außen erstreckt;
**dadurch gekennzeichnet, dass**
der Schaft mindestens eine Stützplatte (82) aufweist, die eine Ringform sowie einen Außendurchmesser aufweist, der komplementär zu einem Außendurchmesser der Grundplatte und im Wesentlichen gleich diesem ist, sich von dem Schaft radial nach außen erstreckt und eine Außenabmessung aufweist, die geringfügig kleiner als ein Innendurchmesser des Spritzenzylinders ist, um ein minimales Spiel gegenüber einer Innenfläche des Spritzenzylinders bereitzustellen und die Kolbenstange zu stabilisieren, wenn diese in dem Spritzenzylinder aufgenommen ist, wobei die Stützplatte von der Grundplatte in Richtung des zweiten Endes axial um einen Abstand beabstandet ist, um die Kolbenstange zu stabilisieren und einem Kippen und einer seitlichen Bewegung der Kolbenstange relativ zum Spritzenzylinder während einer Bewegung der Kolbenstange in einer proximalen Richtung in Bezug auf den Spritzenzylinder entgegenzuwirken und eine Leckage um den Stopfen herum zu hemmen, wenn der Spritzenzylinder mit einer Substanz gefüllt ist und sich die Kolbenstange vor dem Abgeben der Substanz in einer proximalen Position befindet.

2. Kolbenstange nach Anspruch 1, wobei der Spritzenzylinder einen Innendurchmesser aufweist und wobei die Stützplatte von der Grundplatte um einen Abstand beabstandet ist, der etwa dem 1- bis 1,5-Fachen des Innendurchmessers des Spritzenzylinders entspricht.

3. Kolbenstange nach Anspruch 1, wobei der axiale Abstand zwischen der Grundplatte und der Stützplatte 1:1 bis 2:1 relativ zum Innendurchmesser des Spritzenzylinders beträgt.

4. Kolbenstange nach Anspruch 1, wobei die Stützplatte von der Grundplatte um einen Abstand beabstandet ist, bei dem die Stützplatte die Innenfläche des Spritzenzylinders berührt, wenn der Spritzenzylinder vor dem Abgeben mit Inhalt gefüllt ist.

## Revendications

1. Tige de piston configurée pour être utilisée avec un corps de seringue présentant une extrémité proximale ouverte et un passage axial, ladite tige de piston comprenant :
un arbre (52) présentant un axe longitudinal avec une première extrémité et une seconde extrémité opposée à ladite première extrémité, ledit arbre présentant une pluralité de nervures (58) s'étendant vers l'extérieur ;
un organe d'accouplement (62) au niveau de ladite première extrémité présentant un poteau à tête élargie pour l'accouplement à une butée (16), ledit organe d'accouplement présentant une plaque de base configurée pour supporter une extrémité proximale de la butée, et ladite plaque de base présentant une forme annulaire s'étendant radialement vers l'extérieur à partir dudit arbre ;
**caractérisé en ce que**
ledit arbre présentant au moins une plaque de support (82) de forme annulaire et un diamètre externe complémentaire et sensiblement identique à un diamètre externe de ladite plaque de base et s'étendant radialement vers l'extérieur à partir dudit arbre, et présentant une dimension externe légèrement inférieure à un diamètre interne du corps de seringue pour fournir un jeu minimal entre une surface interne du corps de seringue pour stabiliser la tige de piston lorsqu'elle est reçue dans le corps de seringue, ladite plaque de support étant espacée axialement de ladite plaque de base en direction de ladite seconde extrémité d'une distance pour stabiliser et résister à l'inclinaison et au mouvement latéral de ladite tige de piston par rapport au corps de seringue pendant le mouvement de ladite tige de piston dans une direction proximale par rapport au corps de seringue, et pour inhiber les fuites autour de ladite butée lorsque ledit corps de seringue est rempli d'une substance et que ladite tige de piston est dans une position proximale avant la distribution de la substance.

2. Tige de piston selon la revendication 1, dans laquelle le corps de seringue présente un diamètre interne, et dans laquelle ladite plaque de support est espacée de ladite plaque de base d'une distance d'environ 1 à 1,5 fois le diamètre interne du corps de seringue.

3. Tige de piston selon la revendication 1, dans laquelle l'espacement axial entre la plaque de base et la plaque de support est de 1:1 à 2:1 par rapport au diamètre interne du corps de seringue.

4. Tige de piston selon la revendication 1, dans laquelle ladite plaque de support est espacée de ladite plaque de base d'une distance où ladite plaque de support entre en contact avec la surface interne du corps de seringue lorsque le corps de seringue est rempli de contenus avant la distribution.
